(19) Europäisches Patentamt
European Patent Office
Office européen des brevets

(11) **EP 2 347 703 B1**

(12) **EUROPEAN PATENT SPECIFICATION**

(45) Date of publication and mention
of the grant of the patent:
**11.07.2012 Bulletin 2012/28**

(51) Int Cl.:
*A61B 5/00* (2006.01)   *A61B 5/103* (2006.01)
*G01N 21/64* (2006.01)   *A61B 1/00* (2006.01)

(21) Application number: **11150412.2**

(22) Date of filing: **07.01.2011**

(54) **Cancerous or pre-cancerous tissue visualization method and device**

Verfahren und Vorrichtung zur Darstellung von krebsartigem oder vorkrebsartigem Gewebe

Procédé et dispositif de visualisation de tissus cancéreux ou pré- cancéreux

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **21.01.2010 LU 91641**

(43) Date of publication of application:
**27.07.2011 Bulletin 2011/30**

(73) Proprietor: **Centre de Recherche Public - Gabriel
Lippmann**
**4422 Belvaux (LU)**

(72) Inventor: **Orlewski, Pierre**
**9021, ETTELBRUCK (LU)**

(74) Representative: **Office Freylinger**
**P.O. Box 48**
**8001 Strassen (LU)**

(56) References cited:
**WO-A1-2006/069444     WO-A2-02/061405**
**US-A- 5 865 754     US-A1- 2008 212 867**

• **YINGHUA SUN ET AL: "Fluorescence lifetime
imaging microscopy: in vivo application to
diagnosis of oral carcinoma", OPTICS LETTERS,
OSA, OPTICAL SOCIETY OF AMERICA,
WASHINGTON, DC, US, vol. 34, no. 13, 1 July 2009
(2009-07-01), pages 2081-2083, XP001524154,
ISSN: 0146-9592**

EP 2 347 703 B1

## Description

### Technical field

The present invention relates to the field of medical imaging, more particularly to the imaging and visualizing of cancerous or pre-malignant tissue.

### Background Art

Photodynamic Diagnosis (PD) (or Fluorescence Diagnosis (FD)) is a low-invasive method for optical biopsy of the tissue. Prior to PD, the tissue area under investigation is sensitized by exogenous, externally applied photosensitizer, most usually applied under the form of ointment containing δ-aminolevulinic acid (5-ALA) or one of its derivatives. 5-ALA is one of the precursors of protoporphyrin IX (PpIX), an endogenous fluorophore intervening in heme synthesis. It is known that external application of 5-ALA will lead to excess of endogenously produced PpIX with regard to its normal (low) concentration in the tissue. Due to different physiological, morphological and biochemical factors among which the high vesicularizsation and different enzymatic activity are the dominant, PpIX will rather accumulate in pre-malignant and in tumour cells than in normal, healthy tissue. It maybe relatively easily visualized, just by illuminating investigated area with blue light from so-called Soret band (~370 - 408nm), which excites PpIX and causes it to fluoresce with fluorescence peaks at 630 and at 710 nm. Therefore, under a Wood's lamp, surface-located non-melanoma tumors and pre-malignancies will appear as reddish areas.

Today, PD yields only qualitative information. Its major drawbacks are that a) it is not possible to solve the fluorescence spectra only in frequency domain due to contribution from different fluorophores, b) the exciting light from Soret band does not penetrate deep enough into tissue (which raises problems with size estimation e.g. of nodular basal cell carcinoma (BCC)), and c) the (contrast) ratio between the fluorescence intensities of "pathological" and "normal" tissue is not constant. Indeed, this ratio is strongly affected by a large number of endogenous or external detection factors. For example, the achievable contrast strongly depends on the precise chemical type of prodrug (photosensitizer) employed. In the case of basal cell carcinoma, the ratio expected for 5-ALA is 2:1, whereas methyl ester of 5-ALA (methyl-ALA) may yield a ratio of 9:1 [C.Fritsch et col., Photochem. Photobiol. 1998; 68(2): 218-221]. Cellular uptake of photosensitizer and the efficiency of its conversion to the endogenous PpIX are among principal factors since the fluorescence intensity strongly depends on the concentration of fluorophore in the tissue. The latter will obviously be influenced by the quantity and concentration of topically applied prodrug and the time between the application and the fluorescence measurement. Other factors influencing on fluorescence intensity are temperature, individual characteristics of the skin and even oxygenation. The morphological sub-types of the tumor will also bias detectable contrast ratio, which may be demonstrated with nodular, infiltrative or multifocal superficial subtypes of the basal cell carcinoma. As PpIX exposed to the light will be progressively subjected to photobleaching (conversion of PpIX to photoproducts), the illumination wavelength, intensity and exposure time are also to be considered. The fluorescence intensity furthermore depends on scattering, viewing angle, detection distance and the characteristics of the detection optics. Finally, the locally varying levels of auto-fluorescence of the tissue will dramatically influence the discrimination contrast.

A conventional PD method uses a Wood's lamp, i.e. a UVA lamp the emission of which is filtered down to the 320 - 400 nm range and usually peaks at 350 - 360 nm. Band-pass filtering is typically achieved using a so-called Wood's glass - a barium-sodium-silicate glass stained with nickel oxide. A Wood's lamp is used in diagnosis of some fungal infections of the skin and efficiently reveals the presence of porphyrins. Obviously, the image seen in blue light is "polluted" by fluorescence emitted from other endogenous fluorophores, which results in a poor detection contrast. This is a purely qualitative method pretty useful in the first screening but failing in attempt to more precise delimitation of skin pathologies. The contrast may be so weak than in several cases even a well-trained expert cannot succeed with discrimination of affected skin portions.

An imaging system that uses fluorescence intensity is marketed by Biocam GmbH under the name of DyaDerm. That system employs a 12-bit CCD camera. A xenon light source delivers short, sub-100-ms light flashes in the 370-440 nm range. Two types of images of the area to be examined may be recorded: a fluorescence intensity image and a normal colour image. The images may be overlaid and represented in false colours to highlight the cancerous or pre-cancerous tissue. Using 5-ALA photosensitizer and the DyaDerm camera system, T.Gambichler et coll. [Photodermatology, Photoimunology and Photomedicine, 2008, 24, 67-71] set red / green fluorescence (i.e. autofluorescence) contrast ratio to 1.37 and employed this as the only discrimination feature for basal cell carcinoma (BCC) detection and delimitation. Despite reaching a good correlation of the BCC detection, they observed that the estimated mean tumor area was found significantly smaller (97.9 versus 124.5 mm$^2$) than as per clinical examination.

The article "Fluorescence lifetime imaging microscopy: in vivo application to diagnosis of oral carcinoma", by Yinhua Sun et al., Optics Letters, July 1, 2009, Vol. 34, No 13, pp. 2081-2083 discloses a FLIM system allowing displaying both lifetime and intensity information of a carcinoma tumor with surrounding normal tissue.

Document US 2008/0212867 discloses a method and a system for detecting detecting, locating, and characterizing tumors via nonlinear optical imaging techniques capable of characterizing fluorescence intensities and fluorescent lifetime parameters from endogenous flavin adenine dinucleotide (FAD) fluorophors present in a test tissue sample.

**Technical problem**

It is an object of the present invention to provide for improved visualization of cancerous or pre-cancerous tissue. This object is achieved by a method as claimed in claim 1.

**General Description of the Invention**

According to the invention, the cancerous or pre-malignant tissue visualization method comprises the following steps:

○ providing a fluorescence intensity image of a tissue to be examined comprising healthy tissue and cancerous or pre-cancerous tissue, the fluorescence intensity image comprising a first array of pixels, each of which contains a fluorescence intensity value;

○ providing a fluorescence lifetime image of the tissue, the fluorescence lifetime image comprising a second array of pixels, each of which contains a fluorescence lifetime value;

○ localizing a region of healthy tissue and a region of cancerous or pre-malignant tissue in the fluorescence intensity image;

○ identifying in the fluorescence lifetime image one or more first pixels corresponding to the region of healthy tissue and one or more second pixels corresponding to the region of cancerous or pre-malignant tissue;

○ defining a (preferably monotonous) weighting function mapping fluorescence lifetime values onto weighting factors in a range from a predetermined low weighting factor to a predetermined high weighting factor based upon the constraint that the weighting function maps a fluorescence lifetime value contained in the one or more first pixels onto the low weighting factor and a fluorescence lifetime value contained in the one or more second pixels onto the high weighting factor;

○ producing a weighted fluorescence intensity image by weighting the fluorescence intensity values contained in the pixels of the fluorescence intensity image with weighting factors obtained by evaluation of the weighting function at the fluorescence lifetime values contained in corresponding pixels of the fluorescence lifetime image;

○ displaying the weighted fluorescence intensity image.

As will be appreciated, the present method relies not only upon fluorescence intensity to visualize cancerous or pre-malignant tissue but takes into account fluorescence lifetime as well. Fluorescence lifetime is in principle independent from intensity and therefore may serve as an additional discriminator to distinguish healthy from cancerous or pre-malignant tissue. For instance, a single decay fluorescent lifetime of PpIX is substantially higher than lifetimes of other endogenous fluorophores contributing to the observable overall fluorescence. The literature (e.g. K. Konig, J. Biophoton [2008], 1, [1]: 13-23) indicates that deep-tissue PpIX has a fluorescence lifetime ($\tau$) of at least 10 ns, whilst other natural fluorophores and not related to cellular malignancy, excitable at same wavelengths will display much shorter lifetime typically below 3 ns (free and protein-bound NAD(P)H, elastin, collagen) or 5.2 ns (flavines). Therefore, one may assume as a general rule that the higher or lower the measured (overall) fluorescence lifetime is, the higher or lower, respectively, is also the relative concentration of PpIX in the mixture of fluorophores.

It should be noted that the present method not only juxtaposes the fluorescence intensity image and the fluorescence lifetime image but provides an enhanced fluorescence intensity image, wherein the intensity values are weighted according to the corresponding lifetime values. However, separate or juxtaposed displaying of the fluorescence intensity image and the fluorescence lifetime image may be optional steps of the method.

According to a preferred embodiment of the invention, the visualization method comprises, prior to providing the fluorescence intensity image and the fluorescence lifetime image of the tissue, the steps of

○ illuminating the tissue to be examined with excitation radiation, and

○ detecting fluorescence light emitted by the excited tissue in response to the illumination,

As indicated by the claims, protection for these steps is claimed inasmuch as they are not carried out as surgical steps practised on the human or animal body. In the context of the present document, a surgical step comprises, in particular, an invasive step representing a substantial physical intervention on the body, which requires professional medical expertise to be carried out and which entails a substantial health risk even when carried out with the required professional care and expertise.

Preferably, the tissue is illuminated with intensity-modulated (i.e. pulsed) excitation radiation so that the fluorescence light fluoresced by the illuminated tissue is also intensity-modulated. The fluorescence lifetime image may thus be recorded with an imager capable of detecting the phase shift between the modulation of the fluorescence light and the modulation of the excitation radiation. Such phase shift ($\phi$) indicates fluorescence lifetime ($\tau$) via the relationship:

$$\tau=\tan(\phi)/(2\pi\omega)$$

where $\omega$ represents the modulation frequency. The fluorescence lifetime is preferably measured using a so-called lock-in imager, such as described, for instance in the paper "All Solid-State Lock-In Imaging for Wide-Field Fluorescence Lifetime Sensing", by Esposito A. et al., Optics Express 9812, Vol. 13, No. 24, which is herewith incorporated herein by reference in its entirety for those countries in which the legislation permits such incorporation by reference.

The localization of a region of healthy tissue and a region of cancerous or pre-malignant tissue may comprise displaying the fluorescence intensity image, and receiving localization information on the region of healthy tissue and region of cancerous or pre-cancerous tissue via user interaction. In other words, it may be left to the user to select the regions of healthy and cancerous or pre-cancerous tissue in the fluorescence intensity image that shall serve as a basis for the calculation of the weighting function. In this case, the user may be prompted to select regions, which he is sure are healthy and cancerous or pre-cancerous, respectively. Alternatively, the localization of a region of healthy tissue and a region of cancerous or pre-malignant tissue in the fluorescence intensity image may be carried out in an automated fashion based upon the fluorescence intensity values contained in the pixels of the fluorescence intensity image. For instance, the region displaying the highest fluorescence intensity values could be chosen as the region of cancerous or pre-malignant tissue. Similarly - if necessary after identification and discarding of deemed necrotic regions - the region displaying the lowest fluorescence intensity values could be chosen as the region of healthy tissue. As those skilled will appreciate, the regions of healthy tissue and cancerous or pre-cancerous tissue, respectively, serve the purpose of calibration of the weighting function. It may be noted, with respect to the case where user selects these calibration regions, that the user normally knows at least one spot of cancerous or pre-malignant tissue (what is unknown to him is typically the exact boundary of that tissue) and at least one spot of healthy tissue (typically at some safe distance from the cancerous or pre-cancerous spot).

Preferably, the step of detecting the fluorescence light comprises providing the fluorescence intensity image of the tissue by recording it with a CMOS or CCD imager. Such CMOS or CCD imager should preferably be optimized for detecting the fluorescence wavelengths of PpIX. Ideally, the imager should have a quantum efficiency of above 50 % in the wavelength range 600-700 nm and/or a dynamic range of at least 8 bit (more preferably at least 12 bit). The step of detecting the fluorescence light preferably also comprises providing the fluorescence lifetime image of the tissue by recording it with a (e.g. CMOS/CCD) lock-in imager synchronized with the excitation radiation.

More preferably, detecting the fluorescence light comprises recording a plurality of primary fluorescence intensity images of the tissue with a CMOS or CCD imager, the fluorescence intensity image (i.e. the one from which the weighted fluorescence intensity image is produced) being computed from a combination of the primary fluorescence intensity images. The plurality of primary fluorescence intensity images could e.g. be recorded when the tissue is illuminated with excitation radiation at different wavelengths and/or by using different filters to filter the fluorescence light. Detecting the fluorescence light may also comprise recording a plurality of primary fluorescence lifetime images of the tissue with a lock-in imager synchronized with the excitation radiation, the fluorescence lifetime image being computed from a combination of the primary fluorescence lifetime images. The plurality of primary fluorescence lifetime images could e.g. be recorded when the tissue is illuminated with excitation radiation at different wavelengths and/or by using different filters to filter the fluorescence light. It should be noted that the terms "primary fluorescence intensity image" and "primary fluorescence lifetime image" are used here to designate images as recorded by the respective detector, prior to any image processing like contrast enhancement, combination of two or more primary images etc., resulting in one ore more "processed images". Nevertheless, the primary images and the processed images are preferably of the same type, such as e.g. bitmap.

If separate imagers are used to provide the fluorescence intensity images and the fluorescence lifetime images, respectively, the fluorescence intensity image may have a higher imager resolution than the fluorescence lifetime image. This means that one spot in the imaged area may be imaged onto a higher number of pixels by the CMOS or CCD imager than by the lock-in imager. In this case, there is no one-to-one correspondence between the pixels of the intensity image and those of the lifetime image, which one would ideally have in the case of identical image resolutions and identical imaged areas. Accordingly the producing of a weighted fluorescence intensity image comprises: for each pixel of the fluorescence intensity image, determining a corresponding pixel in the fluorescence lifetime image, evaluating the weighting function at the fluorescence lifetime value contained in the determined corresponding pixel to find a weighting factor and weighting the fluorescence intensity value contained in the pixel of the fluorescence intensity image with the found weighting factor.

Preferably, the visualization method according to the invention comprises (non-surgically) applying ointment containing a protoporphyrin IX precursor, e.g. δ-aminolevulinic acid or a derivative thereof (e.g. methyl ALA), onto the tissue.

An aspect of the invention concerns a medical imaging device configured to visualize cancerous or pre-cancerous tissue according to the aforementioned method. Such imaging device comprises

○ an illumination unit configured to emit excitation radiation onto tissue to be imaged;

○ a CCD or CMOS imager for recording a fluorescence intensity image of the tissue to be imaged, the fluorescence intensity image comprising a first array of pixels, each of which contains a fluorescence intensity value;

○ a (CMOS/CCD) lock-in imager for recording a fluorescence lifetime image of the tissue to be imaged, the fluorescence lifetime image comprising a second array of pixels, each of which contains a fluorescence lifetime value;

○ a processor connected to the CCD or CMOS imager and to the CMOS/CCD lock-in imager, the processor being configured

- to localize (autonomously or via interaction with a user) a region of healthy tissue and a region of cancerous or pre-cancerous tissue in the fluorescence intensity image,

- to identify in the fluorescence lifetime image one or more first pixels corresponding to the region of healthy tissue and one or more second pixels corresponding to the region of cancerous or pre-malignant tissue,

- to define a (preferably monotonous) weighting function mapping fluorescence lifetime values onto weighting factors in a range from a predetermined low weighting factor to a predetermined high weighting factor based upon the constraint that the weighting function maps a fluorescence lifetime value contained in the one or more first pixels onto the low weighting factor and a fluorescence lifetime value contained in the one or more second pixels onto the high weighting factor and

- to produce a weighted fluorescence intensity image by weighting the fluorescence intensity values contained in the pixels of the fluorescence intensity image with weighting factors obtained by evaluation of the weighting function at the fluorescence lifetime values contained in corresponding pixels of the fluorescence lifetime image; and

○ a display connected to the processor to display the weighted fluorescence intensity image.

The medical imaging device may e.g. be used to visualize surface-located non-melanoma skin tumours like Basal cell carcinoma, (BCC) and squamous cell carcinoma (SQC) or pre-malignant dysplasia and neoplasia like Bowens disease (BD) or actinic keratosis. It may also be used in examinations of gynaecological and of lower genital tract pathologies and diseases [cf. Hillemans P. et al. 2008], such as cervical intraepithelial neoplasia (GIN) [cf. Hillemans P, Weingardt H., et al., 2000, Andrejevic-Blant S. et al. 2004 ; Hillemans P. et al. 2009], vulvar intraepithelial neoplasia (VIN), [cf. Hillemans P. et al.,2009, Nowakowski Z. et al., 2005] and HPV-related condyloma acuminate, [cf. Fehr MK et al., 2002]. When additionally employing an endoscopic or laparoscopic interface (with the illumination unit, the CCD or CMOS imager and the lock-in imager), the medical imaging device may be applied to visualize lower genital tract tumours like ovarian carcinoma metastases [cf. Löning M. et al., 2004], squamous intraepithelial lesion (SIL) of the cervix [cf. Collinot P. et al., 2006]. Finally, carcinoma in situ (CIS) of the bladder [cf. Jocham D. et al., 2008; Jichlinski P. and Jacquemin D., 2008; Cauberg E. et al. 2009], other cancers like prostate cancer, penile carcinoma, kidney tumours and urethral condyloma may be visualized using this medical imaging device. The medical imaging device may also be used to provide visual feedback in the context of microsurgery of brain malignant gliomas [cf. Stummer W. et al., 2006].

**Brief Description of the Drawings**

Further details and advantages of the present invention will be apparent from the following detailed description of not limiting embodiments with reference to the attached drawings, wherein:

Fig. 1 is a schematic view of a medical imager for cancerous or pre-cancerous tissue visualization;

Fig. 2 is a flow chart illustrating a cancerous or pre-cancerous tissue visualization method according to the invention;

Fig. 3 is a flow chart illustrating a variant of the method of Fig. 2;

Fig. 4 shows graphs of weighting functions as may be used in the present invention;

**Description of Preferred Embodiments**

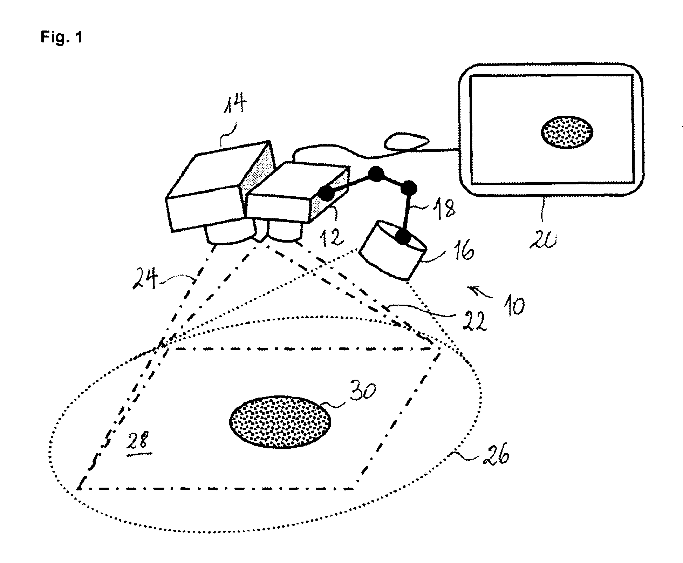

A medical imaging device 10 for cancerous or pre-cancerous tissue visualization is shown in Fig. 1. The imaging device 10 comprises a hand-held unit with a CCD or CMOS camera 12 for recording fluorescence intensity images and a CCD/CMOS lock-in imager 14 for detecting fluorescence lifetime images. The CCD or CMOS camera 12 and the CCD/CMOS lock-in imager 14 are arranged in such a way that their respective fields of view 22, 24 substantially overlay and coincide. The imaging device 10 further comprises an illumination unit 16, which is fixed to the housing of the CCD or CMOS camera 12 and the CCD/CMOS lock-in imager 14 with a hinged joint 18, a processor (not shown, integrated into the housing of the imagers 12, 14) and a display device 20.

The CCD or CMOS camera 12 exhibits a resolution of at least 1200 x 800 pixels, a quantum efficiency of above 50% in the 600-700 nm range and 12 bit or higher dynamic range. The CCD/CMOS lock-in imager 14 typically exhibits substantially lower resolution than the CCD or CMOS camera 12. For technical reasons, the resolution of the CCD or CMOS camera 12 is limited today to maximum 300 x 200 pixels with a quantum efficiency above 60% in similar radiation range.

Both imagers 12 and 14 are individually equipped with dedicated optics, ensuring similar effective fields of view. They are furthermore equipped with filters to selectively block the illumination light. The filters may be changed manually or automatically by means e.g. of a motorised filter wheel or the like.

The illumination unit 16 is controlled to emit intensity-modulated (pulsed) light at least in the Soret band that corresponds to the maximum absorption of PpIX (i.e. at about 403 nm). The CCD/CMOS lock-in imager 14 is synchronized with the illumination unit and detects the phase shift between the modulation of the emitted fluorescence light and the modulation of the excitation light. The phase detection principle used by the imager is the same as the one employed in so-called 3D time-of-flight cameras and is explained in the aforementioned paper by Esposito et al. For each pixel, the lock-in imager 14 thus measures a phase shift ($\phi$) from which the fluorescence lifetime ($\tau$) may be computed as $\tau=\tan(\phi)/(2\pi\omega)$. For improved synchronism of the CCD/CMOS lock-in imager 14 with the illumination unit, some of the pixels of the lock-in imager may be directly coupled via a light guide to the illumination unit and shielded against the light coming from the imaged scene. These pixels thus provide a reference phase with respect to which the phase of the fluorescence light may be calculated.

The excitation light pulses of the illumination unit are generated by solid-state, semiconducting light emitters like LEDs or VCELS. Their number is selected in the way to reach at least a predefined fluence, selected preferably in the range from 0.5 to 15 mW/cm$^2$ and optimally at 2.0 mW/ cm$^2$ at skin plane. The LEDs or VCELS are distributed in such a way as to provide a homogenous distribution of the light at detection plane. To achieve this as well as to adjust the size of the illuminated area 26, a light shaping collimator may be arranged on top of the LEDs or VCSELs. The modulation frequency is preferably selected in the frequency range from 13 kHz to 40 MHz.

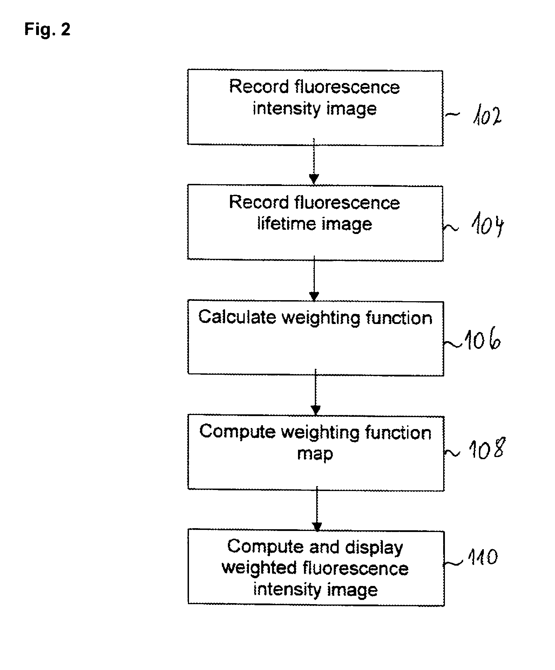

A preferred embodiment of the method according to which the device of Fig. 1 may operate is illustrated in Fig. 2. After the cancerous or pre-cancerous tissue has been found by conventional, optical inspection at ambient light or under a Wood's lamp, photosensitizer (e.g. 5-ALA) is applied to the area to be examined. After the prescribed time has lapsed, the tissue to be examined is illuminated with excitation radiation from the illumination unit 16 and the fluorescence light is detected by means of the imagers 12 and 14. The CCD or CMOS camera 12 records a fluorescence intensity image of the tissue (step 102), while the lock-in imager 14 records a fluorescence lifetime image of the tissue (step 104).

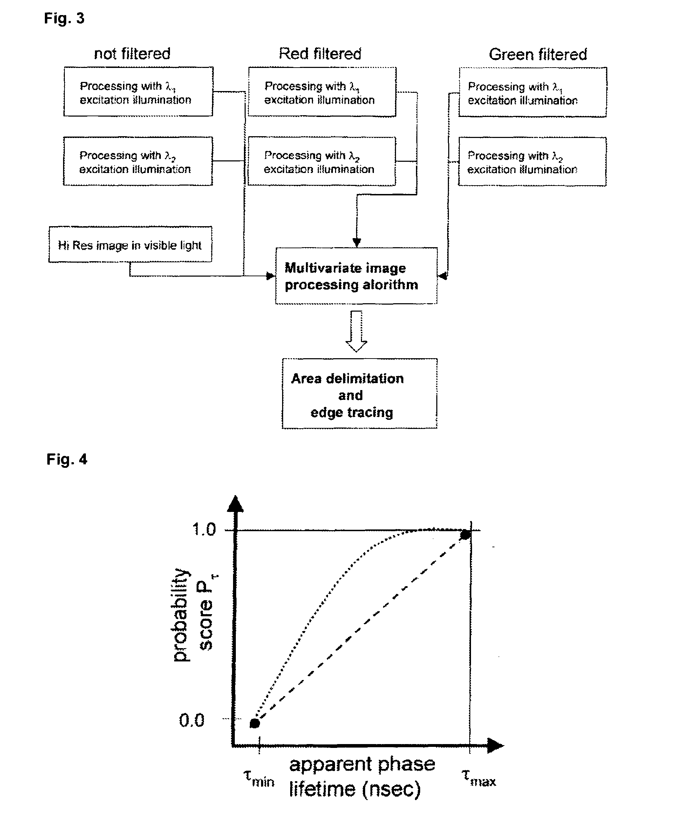

In the next step 106, the processor (which may e.g. be an application-specific integrated circuit, a field-programmable gate array, a digital signal processor or the like) now localizes a region 28 of healthy tissue and a region 30 of cancerous or pre-cancerous tissue in the fluorescence intensity image. It may e.g. achieve this by first eliminating those regions from consideration, in which the fluorescence intensity is locally abnormally low or even null (indicating necrotic tissue), and then setting those pixels with the highest intensity values and those pixels with the lowest intensity values as the regions of cancerous or pre-cancerous tissue and the region of healthy tissue, respectively. The processor then identifies one or more first pixels corresponding to the region of healthy tissue and one or more second pixels corresponding to the region of cancerous or pre-cancerous tissue in the fluorescence lifetime image. In the fluorescence intensity image and the fluorescence lifetime image, "corresponding" pixels are pixels containing information about the same imaged spot. The correspondence could be a one-to-one correspondence in case of identical image resolutions and perfect superposition of the fields of view. The general case is, however, that there is at least a parallax error between the two images and additionally also a difference in the image resolutions. The processor further defines a weighting function mapping fluorescence lifetime values onto weighting factors in a range from a predetermined low weighting factor (e.g. 0) to a predetermined high weighting factor (e.g. 1) based upon the constraint that the weighting function maps a fluorescence lifetime value contained in the one or more first pixels onto the low weighting factor and a fluorescence lifetime value contained in the one or more second pixels onto the high weighting factor. Graphs of possible weighting functions are shown in Fig. 4. The dashed graph corresponds to a linear weighting function (given by $y(\tau)=(\tau-\tau_{min})/(\tau_{max}-\tau_{min})$), whereas the dotted graph corresponds to a monotonously growing non-linear weighting function.

The processor then generates a map (having the image resolution of the fluorescence intensity image) wherein each pixel contains the weighting factor obtained by evaluation of the weighting function at the fluorescence lifetime values contained in corresponding pixels of the fluorescence lifetime image (step 108).

A weighted fluorescence intensity image is then computed by weighting (multiplying) the fluorescence intensity values contained in the pixels of the fluorescence intensity image with the weighting factors (ranging from 0 to 1) contained in the corresponding pixels of the weighting map.

The so-obtained weighted fluorescence intensity image is finally displayed on display 20 (step 110).

According to a preferred embodiment of the method (schematically illustrated in Fig. 3), several fluorescence intensity and lifetime images are recorded. During a first period, the tissue to be examined is illuminated with excitation radiation at a first wavelength (selected in the range from 370 to 430 nm, preferably at about 408 nm). During the first period, three fluorescence intensity images of the tissue are recorded. One of these images is recorded using no filter, another of these images is recorded using a filter having a transmission window from 320 to 550 nm, whereas the third of these images is recorded using a filter having a transmission window from 610 to 750 nm. During a second period, the tissue to be examined is illuminated with excitation radiation at a second wavelength (selected in the range from 500 to 600 nm, preferably at about 530 nm). During the second period, fluorescence intensity and lifetime images are again recorded after delay time T, preferably of few minutes using the abovementioned filters.

The processor combines the primary fluorescence intensity images into a processed fluorescence intensity image. By morphing, warping and/or stretching, all three images are brought to exactly the same reference system. For every image pixel found in the first intensity image (no filter), the radiant background is estimated, for example by fitting pixel intensity slopes with respectively second (320-550 nm) and third (610-750 nm) image pixels.

The processor combines the primary fluorescence lifetime images into a processed fluorescence lifetime image. After subtraction of the radiant background, an artificial image is created based on all images collected during the first and the second period. The processor combines these images in such a way that the intensity of every pixel of the artificial image is given as linear or second order combination of intensities from all collected images. In the following, A1 designates the (relative) weight of the image recorded during the first period without a filter, B1 designates the (relative) weight of the image recorded during the first period with a filter having a transmission window from 320 to 550 nm and C1 designates the (relative) weight of the image recorded during the first period with a filter having a transmission window from 610 to 750 nm. A2 designates the (relative) weight of the image recorded during the second period (after delay T) without a filter, B2 designates the (relative) weight of the image recorded during the second period with a filter having a transmission window from 320 to 550 nm and C2 designates the (relative) weight of the image recorded during the second period with a filter having a transmission window from 610 to 750 nm. The constant parameters A1, B1, C1, A2, B2 and C2 of this combination may be selected in such a way that the combination raises those intensities collected at higher wavelengths (for example by setting A1 = 1, B1=1.5 and C=2). Such parameter selection takes into account the fact that the higher is the fluorescence wavelength, the bigger is the probability that the measured light intensity originates from PpIX and not from another chromophore present in the skin and not related to potential skin malignancy. Alternatively or additionally, the constant parameters may be selected to account for the change (reduction) of the intensities of the images within the same filter configuration (without filter, filter with transmission window from 320 to 550 nm and filter with a transmission window from 610 to 750 nm, respectively) but collected during the first period and the second period, separated by the delay time T. Indeed, the higher is the pixel intensity loss observed (due to fluorophore photobleaching) from the first to the second period, the bigger is the likelihood that the light emission came from malignant tissue (because the concentration of PpIX fluorophore is higher in such tissue, which furthermore lies relatively close to the skin surface and is, therefore, more exposed to the incident light). Preferably, the 3x2 matrix of coefficients A1, B1, C1, A2, B2, and C2 is set and validated experimentally.

The processor now localizes a region of healthy tissue and a region of cancerous or pre-cancerous tissue in the processed fluorescence intensity image. It may e.g. achieve this by first eliminating those regions from consideration, in which the processed fluorescence intensity is locally abnormally low (indicating necrotic tissue), and then setting those pixels with the highest processed intensity values and those pixels with the lowest processed intensity values as the regions of cancerous or pre-cancerous tissue and the region of healthy tissue, respectively. In the next step, the processor identifies one or more first pixels corresponding to the region of healthy tissue and one or more second pixels corresponding to the region of cancerous or pre-cancerous tissue in the processed fluorescence lifetime image.

The processor defines a weighting function mapping processed fluorescence lifetime values onto weighting factors in a range from a predetermined low weighting factor (here: 0) to a predetermined high weighting factor (here: 1) based upon the constraint that the weighting function maps a processed fluorescence lifetime value contained in the one or more first pixels onto the low weighting factor and a processed fluorescence lifetime value contained in the one or more second pixels onto the high weighting factor. The processor then generates a map (having the image resolution of the fluorescence intensity images) wherein each pixel contains the weighting factor obtained by evaluation of the weighting function at the processed fluorescence lifetime values contained in corresponding pixels of the processed fluorescence lifetime image.

A weighted fluorescence intensity image is then computed by weighting (multiplying) the processed fluorescence intensity values contained in the pixels of the fluorescence intensity image with the weighting factors (ranging from 0 to 1) contained in the corresponding pixels of the weighting map.

The so-obtained weighted fluorescence intensity image is finally displayed on display 20.

## Claims

1. Cancerous or pre-cancerous tissue visualization method, comprising providing a fluorescence intensity image (step 102) of a tissue comprising healthy tissue and cancerous or pre-cancerous tissue, said fluorescence intensity image comprising a first array of pixels, each of which contains a fluorescence intensity value;

   providing a fluorescence lifetime image (step 104) of said tissue, said fluorescence lifetime image comprising a second array of pixels, each of which contains a fluorescence lifetime value;

   localizing a region of healthy tissue and a region of cancerous or pre-cancerous tissue in said fluorescence intensity image;

   identifying in said fluorescence lifetime image one or more first pixels corresponding to said region of healthy tissue and one or more second pixels corresponding to said region of cancerous or pre-cancerous tissue; **characterized by**

   defining a weighting function (step 106) mapping fluorescence lifetime values onto weighting factors in a range from a predetermined low weighting factor to a predetermined high weighting factor based upon the constraint that said weighting function maps a fluorescence lifetime value contained in the one or more first pixels onto said low weighting factor and a fluorescence lifetime value contained in the one or more second pixels onto said high weighting factor;

   producing a weighted fluorescence intensity image (step 108) by weighting said fluorescence intensity values contained in the pixels of said fluorescence intensity image with weighting factors obtained by evaluation of said weighting function at the fluorescence lifetime values contained in corresponding pixels of said fluorescence lifetime image;

   displaying (step 110) said weighted fluorescence intensity image.

2. The visualization method according to claim 1, wherein said localizing of a region of healthy tissue and a region of cancerous or pre-cancerous tissue comprises displaying said fluorescence intensity image, and receiving localization information on said region of healthy tissue and region of cancerous or pre-cancerous tissue via user interaction.

3. The visualization method according to claim 1, wherein said localizing of a region of healthy tissue and a region of cancerous or pre-cancerous tissue in said fluorescence intensity image based upon the fluorescence intensity values contained in the pixels is carried out in an automated fashion based upon the fluorescence intensity values contained in the pixels of said fluorescence intensity image.

4. The visualization method according to any one of claims 1 to 3, comprising, prior to providing said fluorescence intensity image and said fluorescence lifetime image, the steps:

   illuminating said tissue with excitation radiation; and
   detecting fluorescence light emitted by said illuminated tissue in response to said illumination; provided that said steps of illuminating said tissue and detecting said fluorescence light are not carried out as surgical steps practised on the human or animal body.

5. The visualization method according to claim 4, wherein said tissue is illuminated with intensity-modulated excitation radiation so that said fluorescence light fluoresced by said illuminated tissue is also intensity-modulated and wherein said fluorescence lifetime image is recorded with an imager capable of detecting a phase shift between the modulation of the fluorescence light and the modulation of the excitation radiation, said phase shift being indicative of fluorescence lifetime.

6. The visualization method according to claim 4 or 5, wherein detecting said fluorescence light comprises providing said fluorescence intensity image of said tissue by recording it with a CMOS or CCD imager, and wherein detecting said fluorescence light comprises providing said fluorescence lifetime image of said tissue by recording it with a lock-in imager synchronized with said excitation radiation.

7. The visualization method according to claim 4 or 5, wherein detecting said fluorescence light comprises recording a plurality of primary fluorescence intensity images of said tissue with a CMOS or CCD imager, wherein said plurality of primary fluorescence intensity images is preferably recorded when said tissue is illuminated with excitation radiation at different wavelengths, and wherein said fluorescence intensity image is computed from a combination of said primary fluorescence intensity images.

8. The visualization method according to claim 7, wherein said plurality of primary fluorescence intensity images is recorded using different filters to filter the fluorescence light.

9. The visualization method according to any one of claims 4 or 6 to 8, wherein detecting said fluorescence light comprises recording a plurality of primary fluorescence lifetime images of said tissue with a lock-in imager synchronized with said excitation radiation, and wherein said fluorescence lifetime image is computed from a combination of said primary fluorescence lifetime images.

10. The visualization method according to claim 9, wherein said plurality of primary fluorescence lifetime images is recorded when said tissue is illuminated with excitation radiation at different wavelengths.

11. The visualization method according to claim 9 or 10, wherein said plurality of primary fluorescence lifetime images is recorded using different filters to filter the fluorescence light.

12. The visualization method according to any one of claims 1 to 11, wherein said fluorescence intensity image has a higher image resolution than said fluorescence lifetime image, and wherein said producing of a weighted fluorescence intensity image comprises: for each pixel of said fluorescence intensity image, determining a corresponding pixel in said fluorescence lifetime image, evaluating said weighting function at the fluorescence lifetime value contained in the determined corresponding pixel to find a weighting factor and weighting the fluorescence intensity value contained in the said pixel of the fluorescence intensity image with the found weighting factor.

13. The visualization method according to any one of claims 1 to 12, comprising non-surgically applying ointment containing a protoporphyrin IX precursor, e.g. δ-aminolevulinic acid or a derivative thereof, onto said tissue.

14. Medical imaging device (10) configured to visualize cancerous or pre-cancerous tissue, comprising
an illumination unit (16) configured to emit excitation radiation on a tissue to be imaged;
a CCD or CMOS imager (12) for recording a fluorescence intensity image, said fluorescence intensity image comprising a first array of pixels, each of which contains a fluorescence intensity value;
a lock-in imager (14) for recording a fluorescence lifetime image of said tissue, said fluorescence lifetime image comprising a second array of pixels, each of which contains a fluorescence lifetime value;
at least one processor connected to said CCD or CMOS imager (12) and to said lock-in imager (14), said at least one processor being configured

 o to localize a region of healthy tissue and a region of cancerous or pre-cancerous tissue in said fluorescence intensity image,
 o to identify in said fluorescence lifetime image one or more first pixels corresponding to said region of healthy tissue and one or more second pixels corresponding to said region of cancerous or pre-cancerous tissue,
 o to define a weighting function mapping fluorescence lifetime values onto weighting factors in a range from a predetermined low weighting factor to a predetermined high weighting factor based upon the constraint that said weighting function maps a fluorescence lifetime value contained in the one or more first pixels onto said low weighting factor and a fluorescence lifetime value contained in the one or more second pixels onto said high weighting factor and
 o to produce a weighted fluorescence intensity image by weighting said fluorescence intensity values contained in the pixels of said fluorescence intensity image with weighting factors obtained by evaluation of said weighting function at the fluorescence lifetime values contained in corresponding pixels of said fluorescence lifetime image; and

a display (20) connected to said processor to display said weighted fluorescence intensity image.

15. Medical imaging device (10) as claimed in claim 14, comprising an endoscopic or a laparoscopic interface equipped with said illumination unit, said CCD or CMOS imager and said lock-in imager.

**Patentansprüche**

1. Verfahren zur Darstellung von krebsartigem oder vorkrebsartigem Gewebe, aufweisend
Bereitstellen eines Fluoreszenzintensitätsbildes (Schritt 102) eines Gewebes, das gesundes Gewebe und krebsartiges oder vorkrebsartiges Gewebe aufweist, wobei das Fluoreszenzintensitätsbild eine erste Anordnung von Pixeln aufweist, von denen jeder einen Fluoreszenzintensitätswert enthält;
Bereitstellen eines Fluoreszenzlebensdauerbildes (Schritt 104) des Gewebes, wobei das Fluoreszenzlebensdauerbild eine zweite Anordnung von Pixeln aufweist, von denen jeder einen Fluoreszenzlebensdauerwert enthält;
Lokalisieren eines Bereichs von gesundem Gewebe und eines Bereichs von krebsartigem oder vorkrebsartigem Gewebe in dem Fluoreszenzintensitätsbild;
Bestimmen eines oder mehrerer erster Pixel in dem Fluoreszenzlebensdauerbild, die dem Bereich von gesundem Gewebe entsprechen, und eines oder mehrerer zweiter Pixel, die dem Bereich von krebsartigem oder vorkrebsartigem Gewebe entsprechen; **gekennzeichnet durch**
das Definieren einer Gewichtungsfunktion (Schritt 106), die Fluoreszenzlebensdauerwerte auf Gewichtungsfaktoren in einem Bereich von einem vorbestimmten niedrigen Gewichtungsfaktor bis zu einem vorbestimmten hohen Gewichtungsfaktor abbildet, und zwar basierend auf der Beschränkung, dass die Gewichtungsfunktion einen in dem einen oder den mehreren ersten Pixeln enthaltenen Fluoreszenzlebensdauerwert auf dem niedrigen Gewichtungsfaktor abbildet, und einen in dem einen oder den mehreren zweiten Pixeln enthaltenen Fluoreszenzlebensdauerwert auf dem hohen Gewichtungsfaktor abbildet;
Erzeugen eines gewichteten Fluoreszenzintensitätsbildes (Schritt 108) durch Gewichten der in den Pixeln des Fluoreszenzintensitätsbildes enthaltenen Fluoreszenzintensitätswerte mit Gewichtungsfaktoren, die durch Auswertung der Gewichtungsfunktion mit den in den entsprechenden Pixeln des Fluoreszenzlebensdauerbildes enthaltenen Fluoreszenzlebensdauerwerten erhalten werden;
Anzeigen (Schritt 110) des gewichteten Fluoreszenzintensitätsbildes.

2. Darstellungsverfahren nach Anspruch 1, wobei das Lokalisieren eines Bereichs von gesundem Gewebe und eines Bereichs von krebsartigem oder vorkrebsartigem Gewebe das Anzeigen des Fluoreszenzintensitätsbildes und das Empfangen von Lokalisierungsinformationen über den Bereich von gesundem Gewebe und den Bereich von krebsartigem oder vorkrebsartigem Gewebe über eine Benutzerinteraktion aufweist.

3. Darstellungsverfahren nach Anspruch 1, wobei das Lokalisieren eines Bereichs von gesundem Gewebe und eines Bereichs von krebsartigem oder vorkrebsartigem Gewebe in dem Fluoreszenzintensitätsbild basierend auf den in den Pixeln enthaltenen Fluoreszenzintensitätswerten auf Grundlage der in den Pixeln des Fluoreszenzintensitätsbildes enthaltenen Fluoreszenzintensitätswerten automatisiert durchgeführt wird.

4. Darstellungsverfahren nach einem der Ansprüche 1 bis 3, das vor dem Bereitstellen des Fluoreszenzintensitätsbildes und des Fluoreszenzlebensdauerbildes die folgenden Schritte aufweist:

    Beleuchten des Gewebes mit Anregungsstrahlung; und
    Erkennen von durch das beleuchtete Gewebe als Reaktion auf die Beleuchtung ausgestrahltem Fluoreszenzlicht;
    mit der Maßgabe, dass die Schritte des Beleuchtens des Gewebes und des Erkennens des Fluoreszenzlichtes nicht als an dem menschlichen oder tierischen Körper vorgenommene chirurgische Schritte ausgeführt werden.

5. Darstellungsverfahren nach Anspruch 4, wobei das Gewebe mit einer intensitätsmodulierten Anregungsstrahlung beleuchtet wird, so dass das von dem beleuchteten Gewebe fluoreszierte Fluoreszenzlicht auch intensitätsmoduliert wird, und wobei das Fluoreszenzlebensdauerbild mit einem Bildgerät aufgezeichnet wird, das in der Lage ist, eine Phasenverschiebung zwischen der Modulation des Fluoreszenzlichtes und der Modulation der Anregungsstrahlung zu erkennen, wobei die Phasenverschiebung die Fluoreszenzlebensdauer anzeigt.

6. Darstellungsverfahren nach Anspruch 4 oder 5, wobei das Erkennen des Fluoreszenzlichtes das Bereitstellen des Fluoreszenzintensitätsbildes des Gewebes durch dessen Aufzeichnung mit einem CMOS- oder CCD-Bildgerät aufweist, und wobei das Erkennen des Fluoreszenzlichtes das Vorsehen des Fluoreszenzlebensdauerbildes des Gewebes durch dessen Aufzeichnung mit einem mit der Anregungsstrahlung synchronisierten Lock-in-Bildgerät aufweist.

7. Darstellungsverfahren nach Anspruch 4 oder 5, wobei das Erkennen des Fluoreszenzlichtes das Aufzeichnen einer Vielzahl von primären Fluoreszenzintensitätsbildern des Gewebes mit einem CMOS- oder CCD-Bildgerät aufweist, wobei die Vielzahl von primären Fluoreszenzintensitätsbildern vorzugsweise aufgezeichnet wird, wenn das Gewebe mit einer Anregungsstrahlung mit verschiedenen Wellenlängen beleuchtet wird, und wobei das Fluoreszenzintensitätsbild aus einer Kombination der primären Fluoreszenzintensitätsbilder berechnet wird.

8. Darstellungsverfahren nach Anspruch 7, wobei die Vielzahl von primären Fluoreszenzintensitätsbildern unter Verwendung von verschiedenen Filtern aufgezeichnet wird, um das Fluoreszenzlicht zu filtern.

9. Darstellungsverfahren nach einem der Ansprüche 4 oder 6 bis 8, wobei das Erkennen des Fluoreszenzlichtes das Aufzeichnen einer Vielzahl von primären Fluoreszenzlebensdauerbildern des Gewebes mit einem mit der Anregungsstrahlung synchronisierten Lock-in-Bildgerät aufweist, und wobei das Fluoreszenzlebensdauerbild aus einer Kombination der primären Fluoreszenzlebensdauerbildern berechnet wird.

10. Darstellungsverfahren nach Anspruch 9, wobei die Vielzahl von primären Fluoreszenzlebensdauerbildern aufgezeichnet wird, wenn das Gewebe mit Anregungsstrahlung mit verschiedenen Wellenlängen beleuchtet wird.

11. Darstellungsverfahren nach Anspruch 9 oder 10, wobei die Vielzahl von primären Fluoreszenzlebensdauerbildern unter Verwendung von verschiedenen Filtern aufgezeichnet wird, um das Fluoreszenzlicht zu filtern.

**12.** Darstellungsverfahren nach einem der Ansprüche 1 bis 11, wobei das Fluoreszenzintensitätsbild eine höhere Bildauflösung aufweist als das Fluoreszenzlebensdauerbild, und wobei das Erzeugen eines gewichteten Fluoreszenzintensitätsbildes aufweist: für jedes Pixel des Fluoreszenzintensitätsbildes das Bestimmen eines entsprechenden Pixels in dem Fluoreszenzlebensdauerbild, das Auswerten der Gewichtungsfunktion bei dem in dem bestimmten entsprechenden Pixel enthaltenen Fluoreszenzlebensdauerwert, um einen Gewichtungsfaktor zu finden, und das Gewichten des in dem Pixel des Fluoreszenzintensitätsbildes enthaltenen Fluoreszenzintensitätswertes mit dem gefundenen Gewichtungsfaktor.

**13.** Darstellungsverfahren nach einem der Ansprüche 1 bis 12, aufweisend das nicht-chirurgische Aufbringen einer Salbe auf das Gewebe, die einen Protoporphyrin-IX-Vorläufer, z. B. eine δ-Aminolävulinsäure oder ein Derivat davon, enthält.

**14.** Medizinische Abbildungsvorrichtung (10), die dafür konfiguriert ist,
krebsartiges oder vorkrebsartiges Gewebe darzustellen, aufweisend
eine Beleuchtungseinheit (16), die dazu konfiguriert ist, eine Anregungsstrahlung auf ein abzubildendes Gewebe auszustrahlen;
ein CCD- oder CMOS-Bildgerät (12) zum Aufzeichnen eines Fluoreszenzintensitätsbildes, wobei das Fluoreszenzintensitätsbild eine erste Anordnung von Pixeln enthält, von denen jeder einen Fluoreszenzintensitätswert enthält;
ein Lock-in-Bildgerät (14) zum Aufzeichnen eines Fluoreszenzlebensdauerbildes des Gewebes, wobei das Fluoreszenzlebensdauerbild eine zweite Anordnung von Pixeln enthält, von denen jeder einen Fluoreszenzlebensdauerwert enthält; mindestens einen Prozessor, der mit dem CCD- oder CMOS-Bildgerät (12) und dem Lock-in-Bildgerät (14) verbunden ist, wobei der mindestens eine Prozessor dafür konfiguriert ist

• einen Bereich von gesundem Gewebe und einen Bereich von krebsartigem oder vorkrebsartigem Gewebe in dem Fluoreszenzintensitätsbild zu lokalisieren,
• in dem Fluoreszenzlebensdauerbild ein oder mehrere erste Pixel, die dem Bereich von gesundem Gewebe entsprechen, und ein oder mehrere zweite Pixel, die dem Bereich von krebsartigem oder vorkrebsartigem Gewebe entsprechen, zu bestimmen,
• eine Gewichtungsfunktion zu definieren, die Fluoreszenzlebensdauerwerte auf Gewichtungsfaktoren in einem Bereich von einem vorbestimmten niedrigen Gewichtungsfaktor bis zu einem vorbestimmten hohen Gewichtungsfaktor abbildet, und zwar basierend auf der Beschränkung, dass die Gewichtungsfunktion einen in dem einen oder den mehreren ersten Pixeln enthaltenen Fluoreszenzlebensdauerwert auf dem niedrigen Gewichtungsfaktor abbildet und einen in dem einen oder den mehreren zweiten Pixeln enthaltenen Fluoreszenzlebensdauerwert auf dem hohen Gewichtungsfaktor abbildet,
• ein gewichtetes Fluoreszenzintensitätsbild durch Gewichten der in den Pixeln des Fluoreszenzintensitätsbildes enthaltenen Fluoreszenzintensitätswerte mit Gewichtungsfaktoren zu erzeugen, die durch Auswertung der Gewichtungsfunktion mit den in entsprechenden Pixeln des Fluoreszenzlebensdauerbildes enthaltenen Fluoreszenzlebensdauerwerten erhalten werden; und

eine Anzeige (20), die mit dem Prozessor verbunden ist, um das gewichtete Fluoreszenzintensitätsbild anzuzeigen.

**15.** Medizinische Abbildungsvorrichtung (10) nach Anspruch 14, aufweisend
eine endoskopische oder eine laparoskopische Schnittstelle, die mit der Beleuchtungseinheit, dem CCD- oder CMOS-Bildgerät und dem Lock-in-Bildgerät ausgestattet ist.

**Revendications**

**1.** Procédé de visualisation de tissu cancéreux ou précancéreux, comprenant
la délivrance d'une image en intensité de fluorescence (étape 102) d'un tissu comprenant du tissu sain et du tissu cancéreux ou précancéreux, ladite image en intensité de fluorescence comprenant un premier ensemble de pixels, chacun desquels contient une valeur d'intensité de fluorescence ;
la délivrance d'une image en durée de vie de fluorescence (étape 104) dudit tissu, ladite image en durée de vie de fluorescence comprenant un deuxième ensemble de pixels, chacun desquels contient une valeur de durée de vie de fluorescence ;
la localisation d'une région de tissu sain et d'une région de tissu cancéreux ou précancéreux dans ladite image en intensité de fluorescence ;
l'identification dans ladite image en durée de vie de fluorescence d'un ou plusieurs premier(s) pixel(s) correspondant(s) à ladite région de tissu sain et d'un ou plusieurs deuxième(s) pixel(s) correspondant(s) à ladite région de tissu cancéreux ou précancéreux ;

**caractérisé par**

la définition d'une fonction de pondération (étape 106) mettant en correspondance des valeurs de durée de vie de fluorescence avec des facteurs de pondération dans une plage d'un facteur de pondération bas prédéterminé à un facteur de pondération haut prédéterminé sur la base de la contrainte que ladite fonction de pondération met en correspondance une valeur de durée de vie de fluorescence contenue dans l'un ou les plusieurs premier(s) pixel(s) avec ledit facteur de pondération bas et une valeur de durée de vie de fluorescence contenue dans l'un ou les plusieurs deuxième(s) pixel(s) avec ledit facteur de pondération haut ;

la production d'une image en intensité de fluorescence pondérée (étape 108) en pondérant lesdites valeurs d'intensité de fluorescence contenues dans les pixels de ladite image en intensité de fluorescence avec des facteurs de pondération obtenus par une évaluation de ladite fonction de pondération aux valeurs de durée de vie de fluorescence contenues dans des pixels correspondants de ladite image en durée de vie de fluorescence ;

l'affichage (étape 110) de ladite image en intensité de fluorescence pondérée.

2. Procédé de visualisation selon la revendication 1, dans lequel ladite localisation d'une région de tissu sain et d'une région de tissu cancéreux ou précancéreux comprend l'affichage de ladite image en intensité de fluorescence, et la réception d'une information de localisation sur lesdites région de tissu sain et région de tissu cancéreux ou précancéreux par l'intermédiaire d'une interaction d'un utilisateur.

3. Procédé de visualisation selon la revendication 1, dans lequel ladite localisation d'une région de tissu sain et d'une région de tissu cancéreux ou précancéreux dans ladite image en intensité de fluorescence sur la base des valeurs d'intensité de fluorescence contenues dans les pixels est effectuée d'une façon automatisée sur la base des valeurs d'intensité de fluorescence contenues dans les pixels de ladite image en intensité de fluorescence.

4. Procédé de visualisation selon l'une quelconque des revendications 1 à 3, comprenant, avant la délivrance de ladite image en intensité de fluorescence et de ladite image en durée de vie de fluorescence, les étapes :

d'éclairage dudit tissu avec un rayonnement d'excitation ; et

de détection de la lumière de fluorescence émise par ledit tissu éclairé en réponse audit éclairage ;

sous réserve que lesdites étapes d'éclairage dudit tissu et de détection de ladite lumière de fluorescence ne sont pas mises en oeuvre comme des étapes chirurgicales mises en pratique sur le corps humain ou animal.

5. Procédé de visualisation selon la revendication 4, dans lequel ledit tissu est éclairé avec un rayonnement d'excitation modulé en intensité de façon à ce que ladite lumière de fluorescence émise par ledit tissu éclairé soit également modulée en intensité et dans lequel ladite image en durée de vie de fluorescence est enregistrée avec un imageur apte à détecter un décalage de phase entre la modulation de la lumière de fluorescence et la modulation du rayonnement d'excitation, ledit décalage de phase étant indicateur de la durée de vie de fluorescence.

6. Procédé de visualisation selon la revendication 4 ou 5, dans lequel la détection de ladite lumière de fluorescence comprend la délivrance de ladite image en intensité de fluorescence dudit tissu en l'enregistrant avec un imageur CMOS ou CCD, et dans lequel la détection de ladite lumière de fluorescence comprend la délivrance de ladite image en durée de vie de fluorescence dudit tissu en l'enregistrant avec un imageur à verrouillage synchronisé avec ledit rayonnement d'excitation.

7. Procédé de visualisation selon la revendication 4 ou 5, dans lequel la détection de ladite lumière de fluorescence comprend l'enregistrement d'une pluralité d'images en intensité de fluorescence primaires dudit tissu avec un imageur CMOS ou CCD, dans lequel ladite pluralité d'images en intensité de fluorescence primaires est préférablement enregistrée lorsque ledit tissu est éclairé avec un rayonnement d'excitation à différentes longueurs d'ondes, et dans lequel ladite image en intensité de fluorescence est calculée à partir d'une combinaison desdites images en intensité de fluorescence primaires.

8. Procédé de visualisation selon la revendication 7, dans lequel ladite pluralité d'images en intensité de fluorescence primaires est enregistrée en utilisant différents filtres pour filtrer la lumière de fluorescence.

9. Procédé de visualisation selon l'une quelconque des revendications 4 ou 6 à 8, dans lequel la détection de ladite lumière de fluorescence comprend l'enregistrement d'une pluralité d'images en durée de vie de fluorescence primaires dudit tissu avec un imageur à verrouillage synchronisé avec ledit rayonnement d'excitation, et dans lequel ladite image en durée de vie de fluorescence est calculée à partir d'une combinaison desdites images en durée de vie de fluorescence primaires.

10. Procédé de visualisation selon la revendication 9, dans lequel ladite pluralité d'images en durée de vie de fluorescence primaires est enregistrée lorsque ledit tissu est éclairé avec un rayonnement d'excitation à différentes longueurs d'ondes.

11. Procédé de visualisation selon la revendication 9 ou 10, dans lequel ladite pluralité d'images en durée de vie de fluorescence primaires est enregistrée en utilisant différents filtres pour filtrer la lumière de fluorescence.

12. Procédé de visualisation selon l'une quelconque des revendications 1 à 11, dans lequel ladite image en intensité de fluorescence a une résolution d'image plus élevée que ladite image en durée de vie de fluorescence, et dans lequel ladite production d'une image en intensité de fluorescence pondérée comprend : pour chaque pixel de ladite image en intensité de fluorescence, la détermination d'un pixel correspondant dans ladite image en durée de vie de fluorescence, l'évaluation de ladite fonction de pondération à la valeur de durée de vie de fluorescence contenue dans le pixel correspondant déterminé pour trouver un facteur de pondération et la pondération de la valeur d'intensité de fluorescence contenue dans ledit pixel de l'image en intensité de fluorescence avec le facteur de pondération trouvé.

13. Procédé de visualisation selon l'une quelconque des revendications 1 à 12, comprenant l'application non chirurgicale d'une pommade contenant un précurseur de la protoporphyrine IX, par exemple de l'acide δ-aminolévulinique ou un dérivé de celui-ci, sur ledit tissu.

14. Dispositif d'imagerie médicale (10) configuré pour visualiser un tissu cancéreux ou précancéreux, comprenant
une unité d'éclairage (16) configurée pour émettre un rayonnement d'excitation sur un tissu devant être imagé ;
un imageur CCD ou CMOS (12) pour enregistrer une image en intensité de fluorescence, ladite image en intensité de fluorescence comprenant un premier ensemble de pixels, chacun desquels contient une valeur d'intensité de fluorescence ;
un imageur à verrouillage (14) pour enregistrer une image en durée de vie de fluorescence dudit tissu, ladite image en durée de vie de fluorescence comprenant un deuxième ensemble de pixels, chacun desquels contient une valeur de durée de vie de fluorescence ;
au moins un processeur connecté audit imageur CCD ou CMOS (12) et audit imageur à verrouillage (14), ledit au moins un processeur étant configuré

o pour localiser une région de tissu sain et une région de tissu cancéreux ou précancéreux dans ladite image en intensité de fluorescence,
o pour identifier dans ladite image en durée de vie de fluorescence un ou plusieurs premier(s) pixel(s) correspondant(s) à ladite région de tissu sain et un ou plusieurs deuxième(s) pixel(s) correspondant(s) à ladite région de tissu cancéreux ou précancéreux,
o pour définir une fonction de pondération mettant en correspondance des valeurs de durée de vie de fluorescence avec des facteurs de pondération dans une plage d'un facteur de pondération bas prédéterminé à un facteur de pondération haut prédéterminé sur la base de la contrainte que ladite fonction de pondération met en correspondance une valeur de durée de vie de fluorescence contenue dans l'un ou les plusieurs premier(s) pixel(s) avec ledit facteur de pondération bas et une valeur de durée de vie de fluorescence contenue dans l'un ou les plusieurs deuxième(s) pixel(s) avec ledit facteur de pondération haut et
o pour produire une image en intensité de fluorescence pondérée en pondérant lesdites valeurs d'intensité de fluorescence contenues dans les pixels de ladite image en intensité de fluorescence avec des facteurs de pondération obtenus par une évaluation de ladite fonction de pondération aux valeurs de durée de vie de fluorescence contenues dans des pixels correspondants de ladite image en durée de vie de fluorescence ; et

un affichage (20) connecté audit processeur pour afficher ladite image en intensité de fluorescence pondérée.

15. Dispositif d'imagerie médicale (10) selon la revendication 14, comprenant une interface endoscopique ou laparoscopique équipée avec ladite unité d'éclairage, ledit imageur CCD ou CMOS et ledit imageur à verrouillage.

**Fig. 1**

**Fig. 2**

```
┌─────────────────────────────┐
│     Record fluorescence      │
│      intensity image         │ ~ 102
└─────────────────────────────┘
              │
              ▼
┌─────────────────────────────┐
│     Record fluorescence      │
│       lifetime image         │ ~ 104
└─────────────────────────────┘
              │
              ▼
┌─────────────────────────────┐
│  Calculate weighting function │
│                              │ ~ 106
└─────────────────────────────┘
              │
              ▼
┌─────────────────────────────┐
│  Compute weighting function  │
│            map               │ ~ 108
└─────────────────────────────┘
              │
              ▼
┌─────────────────────────────┐
│     Compute and display      │
│   weighted fluorescence      │ ~ 110
│      intensity image         │
└─────────────────────────────┘
```

**Fig. 3**

| not filtered | Red filtered | Green filtered |
|---|---|---|
| Processing with $\lambda_1$ excitation illumination | Processing with $\lambda_1$ excitation illumination | Processing with $\lambda_1$ excitation illumination |
| Processing with $\lambda_2$ excitation illumination | Processing with $\lambda_2$ excitation illumination | Processing with $\lambda_2$ excitation illumination |

Hi Res image in visible light

**Multivariate image processing alorithm**

**Area delimitation and edge tracing**

**Fig. 4**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20080212867 A

**Non-patent literature cited in the description**

- **C.FRITSCH.** *Photochem. Photobiol.,* 1998, vol. 68 (2), 218-221
- *Photodermatology, Photoimunology and Photomedicine,* 2008, vol. 24, 67-71
- **YINHUA SUN et al.** Fluorescence lifetime imaging microscopy: in vivo application to diagnosis of oral carcinoma. *Optics Letters,* 01 July 2009, vol. 34 (13), 2081-2083
- **K. KONIG.** *J. Biophoton,* 2008, vol. 1 (1), 13-23
- **ESPOSITO A. et al.** All Solid-State Lock-In Imaging for Wide-Field Fluorescence Lifetime Sensing. *Optics Express,* vol. 13 (24), 9812